# EUROPEAN PATENT APPLICATION

(11) **EP 1 484 061 A1**
(43) Date of publication of application: **08.12.2004**
(21) Application number: 03744055.9
(22) Date of filing: 11.03.2003
(51) Int. Cl.: A61K 31/675, A61K 9/10, A61K 9/14, A61K 9/16, A61K 9/20, A61K 9/48, A61K 31/525, A61P 1/16, A61P 1/18, A61P 9/00, A61P 9/10, A61P 17/02, A61P 29/00, A61P 37/00, A61P 43/00, C07D 475/14

(54) **DRUGS CONTAINING RIBOFLAVIN-TYPE COMPOUNDS**

(30) Priority: 11.03.2002 JP 2002065720
(71) Applicant: Eisai Co., Ltd., Tokyo 112-8088 (JP)
(72) Inventor: ARAKI, Seiichi, Tsuchiura-shi, Ibaraki 300-0810 (JP); SUZUKI, Mamoru, Tsukuba-shi, Ibaraki 305-0035 (JP); KODAMA, Kohtarou, Tsuchiura-shi, Ibaraki 300-0815 (JP); TOYOSAWA, Toshio, Tsukuba-shi, Ibaraki 305-0041 (JP)
(74) Representative: HOFFMANN EITLE
(86) International application number: PCT/JP2003/002868
(87) International publication number: WO 2003/075935

(57) **Abstract**

A medicament is provided containing as an active ingredient at least one of riboflavin, a riboflavin derivative or a pharmacologically acceptable salt thereof, which has the action of suppressing IL-1β, IL-6, IL-10, INF-γ, TNF-α, GM-CSF, IL-8, MCP-1 and other cytokines. Moreover, a preventative or therapeutic agent for inflammatory diseases accompanied by hypercytokinemia is provided which has excellent cytokine suppression effect.

## Description

### TECHNICAL FIELD

The present invention relates to a medicament containing riboflavin, a riboflavin derivative or a pharmacologically acceptable salt thereof (hereinbelow sometimes referred to as a riboflavin compound) as an active ingredient, and having a cytokine suppression effect. The present invention also relates to a medicament useful for preventing and treating systemic inflammatory response syndrome not caused by an infection.

### BACKGROUND OF ART

Inflammatory reactions are involved in many diseases, and it is known that inflammatory reactions have an important influence not only in so-called inflammatory diseases, but also in Alzheimer's disease, heart disease and the like.

Of the diseases involving inflammatory reactions, systemic inflammatory response syndrome, which has a pathology of especially systemic inflammatory signs, is particularly important as an indicator of a reaction of the body which was subjected to invasion. Once systemic inflammatory response syndrome has occurred, if the symptoms progress or complications occur, there is a risk of developing adult respiratory distress syndrome (ARDS), disseminated intravascular coagulation (DIC), multiple organ failure (MOF) or another multiple organ dysfunction syndrome (MODS), and such symptoms as disturbance of consciousness, breathing difficulties, low blood pressure and the like, thereby leading to shock and in some cases to death. In addition to infection, causes of this systemic inflammatory response syndrome include a variety of kinds of invasion including wounds, burns, pancreatitis, surgery and the like.

It has long been known that the pathology of systemic inflammatory response syndrome involves over-induction of protein signal transmitters called cytokines. In this regard, it has been reported that there is a specific correlation between the progress of systemic inflammatory response syndrome and blood concentrations of cytokines (Ogawa, Michio: New Invasion and Cytokines, a Double-edged Sword Which Protects and Destroys the Body, Medical Sense, Tokyo, 1999; Bone, R.C., "Toward a theory regarding the pathogenesis of the systemic inflammatory response syndrome: What we do and do not know about cytokine regulation," *Crit. Care Med.* 24, 163-172, 1996). From this it could be said that systemic inflammatory response syndrome is a form of hypercytokinemia.

Efforts have already been made to suppress the induction and expression of cytokines in order to prevent or treat hypercytokinemia.

For example, an attempt was made to suppress induction of cytokines by administering an adrenocortical steroid (Luce, J.M. et al: "Ineffectiveness of high-dose methylprednisolone in preventing parenchymal lung injury and improving mortality in patients with septic shock," *Am. Rev. Respir. Dis.* 138, 62-68, 1998).

Attempts have also been made to suppress the functional expression of cytokines by administering an anti-TNF-α antibody, interleukin-1 receptor antagonist (IL-1RA) or the like (Abraham, E. et al: "Efficacy and safety of monoclonal antibody to human tumor necrosis factor α in patients with sepsis syndrome: A randomized, controlled, double-blind, multicenter clinical trial," *J.A.M.A.* 273, 934-941, 1995; Fisher, C.J. et al: "Recombinant human interleukin-1 receptor antagonist in the treatment of patients with sepsis syndrome: Results from a randomized, double-blind, placebo-controlled trial," *J.A.M.A.* 271, 1836-1844,1994; Dhainaut, J-F.A, et al: "Platelet-activating factor receptor antagonist BN 5021 in the treatment of severe sepsis: A randomized, double-blind, placebo-controlled, multicenter clinical trial," *Crit. Care Med.* 22, 1720-1728, 1994; Fisher, J-C.J. et al: "Treatment of septic shock with the tumor necrosis factor receptor: Fc fusion protein," *E. Engl. J. Med.* 334, 1697-1702, 1996; Bone, R.C.: "Sir Isaac Newton, sepsis, SIRS, and CARS," *Crit. Care Med.* 24, 1125-1178, 1996).

An anti-inflammatory drug composition having N-[2-(2-phthalimideethoxy)acetyl]-L-alanyl-D-glutamic acid as an active ingredient which controls cytokine production reactions is also disclosed in Japanese Patent Kokai Publication No. 11-322601.

Moreover, a pharmaceutical composition for blocking production of active TNF-α which contains a carboxylated and/or sulfated oligosaccharide is disclosed in Japanese Patent Kohyo Publication No. 8-506322.

### DISCLOSURE OF INVENTION

However, such preventative or therapeutic agents for hypercytokinemia have not offered sufficient effectiveness in all cases depending on the pathology and progress of the disease, and there is a demand for drugs with greater effectiveness, which are also excellent for improving prognosis.

In Japanese Patent Kokai Publications Nos. 5-201864 and 10-29941 it is disclosed that riboflavin compounds are useful as immune activators, preventatives against infection and preventative and therapeutic agents for toxic shock, but the relationship between riboflavin compounds and cytokines is not disclosed therein.

Consequently, it is an object of the present invention to provide an excellent medicament for the prevention or treatment of hypercytokinemia.

After exhaustive research the inventors discovered that riboflavin compounds have excellent cytokine suppression effect. That is, it was found that the induction and expression of cytokines can be suppressed by administration of a drug containing a riboflavin compound as an active ingredient, with excellent effectiveness in the treatment of various diseases involving inflammatory responses, particularly hypercytokinemia of a body exposed to excessive invasion.

This is thought to be due to the fact that riboflavin compound acts to suppress the induction or expression of inflammatory cytokines and preserve homeostasis of the body when such inflammatory cytokines are induced or expressed in the body due to invasion.

In this way, the novel discovery that the riboflavin compound has a cytokine suppression effect made it possible to provide with the present invention a more effective preventative or therapeutic agent for hypercytokinemia.

Namely, the present invention provides a medicament having a cytokine suppression effect, which has an active ingredient at least one of riboflavin, a riboflavin derivative or a pharmacologically acceptable salt thereof.

In other words, the present invention provides a cytokine suppressor comprising as an active ingredient at least one of riboflavin, a riboflavin derivative or a pharmacologically acceptable salt of these.

The term "comprising as an active ingredient at least one" signifies that any one of riboflavin, riboflavin derivatives and pharmacologically acceptable salts of the foregoing may be an active ingredient solely, or that more than one of the above may be active ingredients in combination.

The medicament according to the present invention is useful in preventing or treating hypercytokinemia. Moreover, the method of preventing or treating hypercytokinemia according to the present invention comprises administering to a subject an effective dose of at least one of riboflavin, a riboflavin derivative and a pharmacologically acceptable salt thereof.

The term "hypercytokinemia" is a condition in which the concentration of cytokines in the blood is elevated, and examples of hypercytokinemia include Alzheimer's disease, Parkinson's disease, Castleman's disease, rheumatoid arthritis, multiple myositis, systemic sclerosis, combined connective-tissue disease, Felty's syndrome, recurrent rheumatism, sarcoidosis, osteoarthritis, multiple sclerosis, Behçet's disease, lupus erythematosus, atherosclerosis, heart disease, atrial myxoma, Crohn's disease, ulcerative colitis, inflammatory colitis, gout, contact dermatitis, autoimmune dermatitis, psoriasis, pulmonary fibrosis, pulmonary emphysema, diffuse panbronchiolitis, pleurisy, glomerulonephritis, acute glomerulonephritis, mesangial nephritis, lupus nephritis, IgA nephritis, diabetic nephritis, atherosclerosis, nodular arteriosclerosis, angiitis, bronchial asthma, chronic gingivitis, periodontal disease, hemorrhagic shock, traumatic shock, anaphylactic and other kinds of shock, bums, brain tumors, malignant tumors, multiple myeloma, osteoporosis, hemorrhoids, atopic dermatitis, allergic rhinitis, allergic dermatitis and other allergies, sepsis, septic shock, multiple organ failure, carbon monoxide poisoning, drug and other poisoning, acute radiation damage, subarachnoid hemorrhage, systemic inflammatory response syndrome, insulin-dependent diabetes, uveitis, chronic inflammation, blood disorders, leukemia, collagen disease, amyotrophic lateral sclerosis, cerebral edema, epilepsy, idiopathic thrombocytopenic purpura, myasthenia gravis, neuralgia, Guillan-Barré syndrome, neuropathy, spinal damage, pollinosis and other autoimmune disorders, cerebral infarction, cardiac infarction and other ischemia-reperfusion injury, arteriosclerosis, chronic cardiac insufficiency, myocarditis, photophobia, empyema, ankylosing spondylitis, Wegner's granulomatosis, multiple myositis and dermatomyositis, Sjögren's syndrome, temporal arteritis, SAPHO syndrome, chronic fatigue syndrome, systemic juvenile idiopathic arthritis, adult Still's disease, endometriosis, uterine empyema, otitis media, peritonitis, endocarditis, acute renal failure, acute hepatopathy, diarrhea and cataracts.

The term "sepsis" used herein refers to sepsis in which blood cytokine concentrations are higher than normal values. Septic shock signifies a condition in which sepsis progresses to the point that dysfunction of one or more internal organs such as the heart, lung, liver, kidneys, spleen, brain or spinal cord occurs, or in which organ dysfunction results in such symptoms as weakness, dizziness, difficulty in standing, low blood pressure, low body temperature, arrhythmia, ventricular fibrillation, dyspnea, low body temperature, convulsions, impaired consciousness or unconsciousness.

Moreover, the medicament according to the present invention is particularly useful for the prevention or treatment of systemic inflammatory response syndrome.

Systemic inflammatory response syndrome is one kind of hypercytokinemia, and is thought to have two pathologies-systemic inflammatory response syndrome (SIRS), in which an inflammatory response occurs when inflammatory cytokines predominate in the blood, and compensatory anti-inflammatory response syndrome (CARS), in which an anti-inflammatory response (such as severe infection) occurs when anti-inflammatory cytokines predominate in the blood, resulting in immunosuppression. It was found that when the medicament according to the present invention which has a riboflavin compound as an active ingredient is administered, it has the effect of preventing or treating SIRS symptoms by its cytokine suppression effect. Moreover, because SIRS and CARS are interrelated together, prevention or treatment for SIRS can also serve to prevent or treat CARS through the cytokine suppression effect of the medicament of the present invention.

The term "prevention or treatment of systemic inflammatory response syndrome" used in the present invention includes prevention or treatment of adult respiratory distress syndrome (ARDS), disseminated intravascular coagulation (DIC), multiple organ failure (MOF) and other multiple organ dysfunction syndromes (MODS), which are caused when the symptoms of SIRS or CARS progress, and the medicament according to the present invention is extremely useful for the prevention or treatment of pathologies of advanced SIRS or CARS.

Moreover, after exhaustive research the present inventors discovered that the medicament according to the present invention which comprises the riboflavin compound as an active ingredient has the particular effectiveness in preventing or treating systemic inflammatory response syndrome not caused by an infection.

Examples of "systemic inflammatory response syndrome not caused by an infection" include systemic inflammatory response syndrome caused by wounds, burns, acute or chronic pancreatitis, alcoholic liver injury, drug-induced liver injury, liver damage, hepatitis, cirrhosis of the liver, appendicitis, ulcers, ischemia/reperfusion injury, type I diabetes, type II diabetes, diabetic deterioration, prevention or treatment of aftereffects of peritoneal dialysis, or surgery.

The aforementioned cytokine suppression effect is preferably action which suppresses at least one of the group consisting of IL-1β, IL-6, IL-10, INF-γ, TNF-α, GM-CSF, IL-8 or MCP-1.

In the present invention, the action of suppressing chemokines, which are a kind of cytokine, is included in the meaning of "cytokine suppression effect". The term "chemokine" means a general term for more than 50 cytokines with a molecular weight of about 10,000 having cysteine residues in conserved positions, which exhibit mobilizing activity towards leukocytes. Examples of cytokines include IL-1β (interleukin-1β), IL-6 (interleukin-6), IL-10 (interleukin-10), INF-γ (interferon-γ), TNF-α (tumor necrosis factor-α) and GM-CSF (granulocyte macrophage-colony stimulating factor), while examples of chemikines (a kind of cytokine) include IL-8 (interleukin-8) and MCP-1 (macrophage chemoattractant protein-1).

The present invention also provides a medicament containing as an active ingredient at least one of riboflavin, a riboflavin derivative or a pharmacologically acceptable salt thereof, which is used in the prevention or treatment of systemic inflammatory response syndrome not caused by an infection.

Examples of "systemic inflammatory response syndrome not caused by an infection" include systemic inflammatory response syndrome caused by wounds, burns, pancreatitis, liver damage, ischemia-reperfusion injury or surgery.

In the aforementioned, the riboflavin derivative or pharmacologically acceptable salt thereof is preferably flavin mononucleotide, flavin adenine dinucleotide, riboflavin tetrabutylate, riboflavin sodium phosphate, riboflavin phosphate monodiethanolamine salt, leucoflavin, monohydroflavin, leucoflavin phosphoric acid ester, leucoflavin mononucleotide, leucoflavin adenine dinucleotide or a pharmacologically acceptable salt thereof.

The aforementioned cytokine suppression effect may have scavenging action of active oxygen as one of its mechanisms. In other words, the cytokine suppression effect of administration of the aforementioned riboflavin compound may have as one of its mechanisms the action of preventing functional damage to tissue by the active oxygen or in other words the action of scavenging the active oxygen.

The targets for administration of the medicament according to the present invention are humans or other animals. The medicament of the present invention is particularly useful for prevention or treatment in humans.

Animals in the present invention are industrial animals, companion animals and experimental animals. Industrial animals are animals which need to be fed for industrial purposes, such as bovines, equines, swines, caprines, ovines and other livestock, chickens, ducks, quail, turkeys, ostriches and other fowl, and yellowtail, young yellowtail, red sea bream, horse mackerel, carp, rainbow trout, eels and other fish. Companion animals are dogs, cats, marmosets, cage birds, hamsters, goldfish and other pet animals, while experimental animals are rats, guinea pigs, beagle dogs, miniature pigs, rhesus monkeys, crab-eating macaques and other animals used for research in medical, biological, agricultural, pharmaceutical and other fields.

There are no particular limitations on the form of administration of the medicament according to the present invention, which differs depending on the pathology and progress of the disease and other conditions, but it is desirable that at least one of the aforementioned riboflavin, riboflavin derivative or pharmacologically acceptable salt thereof be contained in the form of an injection, tablet, granule, powder, subtle granule, capsule, pill or oral liquid (including syrup). From the standpoint of further improving the cytokine suppression effect, administration is preferably by injection.

When administration is in injection form, the administration is preferably intravenous, intraperitoneal, intramuscular, subcutaneous, intradermal, intra-articular, intra-bursal, intra-thecal, intra-periosteal or the like, with intravenous administration or intraperitoneal administration being particularly preferred. Intravenous administration may be either drip administration or bolus administration.

There are no particular limitations on the administered dose (dose of active ingredients), which differs depending on the pathology and progress of the disease and other conditions (species, symptoms, age, weight and sex of the subject of administration, and complications, administration period, administration method, formulation, differences in sensitivity and the like), but when administering an injection by intravenous drip, administration of 0.001 to 10 mg/kg/h for a few minutes to a week is preferred. When administering by intravenous bolus, the administered dose is 0.1 to 50 mg/kg or preferably 0.3 to 20 mg/kg or more preferably 2 to 20 mg/kg. In the case of intraperitoneal administration it is 0.1 to 50 mg/kg or preferably 0.3 to 20 mg/kg or more preferably 2 to 20 mg/kg. In the case of intramuscular administration it is 0.1 to 50 mg/kg or preferably 2 to 20 mg/kg. In the case of oral administration it is 1 to 1000 mg/kg or preferably 10 to 500 mg/kg or more preferably 30 to 200 mg/kg.

The medicament according to the present invention may be administered as is or may be made into an injection (for intravenous administration (drip, bolus), intraperitoneal administration, intramuscular administration, subcutaneous administration or the like), oral form (tablet, granule, powder, subtle granule, capsule, pill, oral liquid, syrup or the like), percutaneous absorption form, eye drop, nose drop, suppositories, inhalants (aerosol, powder inhalant, liquid inhalant or the like) or external form (salve, cream, liquid or the like) by ordinary methods with the addition of commonly used pharmaceutical additives. It can also be mixed with a food, feed, drink or the like.

When manufacturing the injection, manufacture can be by ordinary methods with an addition of a solubilizer, a pH adjuster, a buffer, a suspending agent, an anti-oxidant, a preservative, an isotonic agent and the like to the riboflavin compound as necessary. A drip injection may be formed with a solubilizer or the like as the infusion solution, or a drip injection may be formed with an infusion solution having pharmacologically acceptable modifications added thereto. These injections can be administered intravenously, intraperitoneally, intramuscularly, subcutaneously or the like. Alternatively, they can be free-dried to make freeze-dried preparations which are dissolved as necessary.

When manufacturing oral solid preparations, an excipient, a binder, a disintegrator, a lubricant, a colorant, a flavoring, an anti-oxidant, a solubilizer, a stabilizer and the like can be added to the riboflavin compound as necessary and tablet, coated tablet, granule, powder, subtle granule, capsule, (hard capsule, soft capsule), pill or the like prepared by ordinary methods.

There are no particular limits on the solubilizer used, but examples of the solubilizer include saline, phosphate buffered saline, lactated Ringer's solution, polyoxyethylene hydrogenated castor oil, polysorbate 80, nicotinamide, polyoxyethylene sorbitan monolaurate, macrogol, castor oil fatty acid ethyl ester and the like.

There are no particular limits on the pH adjuster or the buffer, but examples include organic acids or inorganic acids and/or salts thereof as well as sodium hydroxide, meglumine and the like.

There are no particular limits on the suspending agent, but examples include methylcellulose, polysorbate 80, hydroxyethylcellulose, gum arabic, carboxymethylcellulose sodium, polyoxyethylene sorbitan monolaurate, tragacanth powder and the like.

There are no particular limits on the anti-oxidant, but examples include ascorbic acid, α-tocopherol, ethoxyquin, dibutyl hydroxytoluene, butyl hydroxyanisole and the like.

There are no particular limits on the preservative, but examples include methyl para-hydroxybenzoate, ethyl-para-hydroxybenzoate, sorbic acid, phenol, cresol, chlorocresol and the like.

There are no particular limits on the isotonic agent, but examples include sodium chloride and the like.

There are no particular limits on the excipient, but examples include starch, corn starch, dextrin, glucose, lactose, saccharose, sugar alcohols (mannitol, erythritol, xylitol and the like), hydrogenated oil, crystal cellulose, silicic anhydride, calcium silicate, calcium dihydrogenphosphate and the like.

There are no particular limits on the binder, but examples include polyvinylpyrrolidone, ethyl cellulose, methyl cellulose, alpha starch, gum arabic, hydroxypropyl cellulose, hydroxypropyl methylcellulose, carboxymethylcellulose, carboxymethylcellulose sodium, propylene glycol, sodium polyacrylate, polyvinyl alcohol and the like.

There are no particular limits on the disintegrator, but examples include crospovidone, low-substituted hydroxypropyl cellulose, cross-linked carboxymethylcellulose sodium, carboxymethylcellulose, calcium carbonate, carboxymethylcellulose calcium and the like.

There are no particular limits on the lubricant, but examples include magnesium stearate, talc, calcium stearate, sodium stearyl fumarate, polyethylene glycol 6000 and the like.

The tablet, granule or powder can be provided with a coating such as hydroxypropyl methylcellulose as necessary.

When manufacturing an oral liquid, manufacture can be by ordinary methods with the colorant, the flavoring, the anti-oxidant, the solubilizer, the stabilizer and the like added to the riboflavin compound as necessary.

The present invention also provides the use of at least one of riboflavin, a riboflavin derivative or a pharmacologically acceptable salt thereof for preparation of a preventative or therapeutic agent for hypercytokinemia. In other words, the present invention provides a method for manufacturing a preventative or therapeutic agent for hypercytokinemia using at least one of riboflavin, a riboflavin derivative or a pharmacologically acceptable salt thereof.

Moreover, the present invention also provides the use of riboflavin, a riboflavin derivative or a pharmacologically acceptable salt thereof as a preventative or therapeutic agent for hypercytokinemia.

Moreover, the present invention also provides a method for treating diseases (such as inflammatory diseases, hypercytokinemia and systemic inflammatory response syndrome) which require cytokine suppression, wherein at least one of riboflavin, a riboflavin derivative or a pharmacologically acceptable salt thereof is administered to the subject (such as the animal).

### DESCRIPTION OF THE DRAWINGS

Figure 1 is a graph showing the effect of 5'-FMN-Na on plasma levels of 1L-1β concentrations in LPS-induced shock mice.
Figure 2 is a graph showing the effect of 5'-FMN-Na on plasma levels of INF-γ concentrations in LPS-induced shock mice.
Figure 3 is a graph showing the effect of 5'-FMN-Na on plasma levels of IL-6 concentrations in LPS-induced shock mice.
Figure 4 is a graph showing the effect of 5'-FMN-Na on plasma levels of GM-CSF concentrations in LPS-induced shock mice.
Figure 5 is a graph showing the effect of 5'-FMN-Na on plasma levels of IL-10 concentrations in LPS-induced shock mice.
Figure 6 is a graph showing the effect of 5'-FMN-Na on plasma levels of TNF-α concentrations in LPS-induced shock mice.
Figure 7 is a graph showing the effect of 5'-FMN-Na on plasma levels of MCP-1 concentrations in LPS-induced shock mice.
Figure 8 is a graph showing the effect of 5'-FMN-Na on plasma levels of MIP-2 concentrations in LPS-induced shock mice.
Figure 9 is a graph showing the effect of 5'-FMN-Na on plasma levels of NO concentrations in LPS-induced shock mice.
Figure 10 is a graph showing the effect of 5'-FMN-Na on plasma levels of INF-γ concentrations in SEB-induced shock mice.
Figure 11 is a graph showing the effect of 5'-FMN-Na on plasma levels of MIP-2 concentrations in SEB-induced shock mice.
Figure 12 is a graph showing the effect of 5'-FMN-Na on plasma levels of IL-6 concentrations in SEB-induced shock mice.
Figure 13 is a graph showing the effect of 5'-FMN-Na on IL-6 production from TNF-α-stimulated mouse peritoneal macrophage.
Figure 14 is a graph showing active oxygen levels produced by high-concentration glucose load.
Figure 15 is a graph showing IL-8 concentrations induced by high-concentration glucose load.

### DETAILED DESCRIPTION

Next, the present invention is explained in detail using examples, but the present invention is not limited thereby.

### (Materials used in tests)

For the riboflavin-5'-phosphate sodium ester (riboflavin sodium phosphate), Japanese Pharmacopoeia riboflavin phosphate sodium ester (5'-FMN-Na) was synthesized and dissolved in 3 mg/mL saline (Otsuka Pharmaceutical Co., Ltd., Tokyo, Japan) for use.

For the LPS (lipopolysaccharide), lipopolysaccharide derived from *Escherichia coli* serum type 0111:B4 was purchased from Sigma (Sigma Chemical Co., St. Louis, MO) and dissolved in saline for use.

For the ELISA (enzyme-linked immunosorbent assay), kits were purchased from Biosource International (Biosource International Inc., Camarillo, CA, USA) and used for measuring concentrations of IL-1β, IL-6, IL-10, INF-γ, TNF-α, GM-CSF and MCP-1. A kit which purchased from TECHNE (TECHNE Corporation, Minneapolis, MN, USA) was used for measuring MIP-2 concentrations. MIP-2 is a mouse chemokine, and a mobilizing factor for neutrophils. It corresponds to IL-8 in humans.

Kits purchased from Dojindo (Dojindo Laboratories, Kumamoto, Japan) were used as the NO₂/NO₃ assay kits.

*Staphylococcus aureus* enterotoxin B and BT-202 were used for the SEB (*Staphylococcus aureus* enterotoxin).

For the TNF-α, human TNF-α. K051 was purchased from Pepro Tech (Pepro Tech, Inc., Rock Hill, NJ, USA) and dissolved 15 µg/mL in saline (Otsuka Pharmaceutical Co., Ltd., Tokyo, Japan) for use.

For the D-galactosamine, D-galactosamine hydrochloride was purchased from Wako (Wako Pure chemical Industries, Ltd., Osaka, Japan) and dissolved 90 mg/mL in saline (Otsuka Pharmaceutical Co., Ltd., Tokyo, Japan) for use.

The AAPH (2,2'-azobis (2-amidinopropane) dihydrochloride) was purchased from Wako Pure Chemical Industries, and dissolved in saline (18.75 mg/mL) for use. AAPH is a free radical initiator, a water-soluble azo compound which generates free radicals by thermal decomposition.

### [Example 1: Effects on endotoxin-induced shock mouse model]

Male ICR mice aged 5 weeks were obtained from Japan SLC, Inc. (Shizuoka, Japan) and housed at a room temperature of 23 °C (permissible range : 20-26 °C) and a relative humidity of 55 % (permissible range : 40 -70 %) with a 12-hour light/dark cycle (lights on at 7:00 a.m., lights off at 7:00 p.m.). The mice were allowed free access to sterile tap water and a laboratory diet (MF, Oriental Yeast Co., Ltd., Tokyo, Japan). After one week of acclimation, mice were used for experiment.

12 mg/kg of LPS was first administered intravenously to the male ICR mice (aged 6 weeks) to prepare endotoxin-induced shock mice.

In the control group, blood was taken 1 hour, 3 hours, 6 hours, 9 hours, 12 hours, 15 hours, 18 hours, 21 hours and 24 hours after administration of LPS (0 hours). In the 5'-FMN-Na administration group, one intravenous administration of 20 mg/kg of 5'-FMN-Na was given 6 hours after administration of LPS, and blood was taken 9 hours, 12 hours, 15 hours, 18 hours, 21 hours and 24 hours after administration of LPS. At each blood collection, each group consisted of 5 mice. Mice which died during the tests were not used as data. Plastic syringes containing EDTA (ethylene diamine tetraacetic acid) were used for collecting blood, which was taken from abdominal veins. Plasma was isolated from the collected blood by centrifugation (3000 rpm, 10 minutes, 4°C), and stored at -80°C.

Next, the cytokine concentrations, chemokine concentrations and NO (nitrogen monoxide) concentrations of the collected plasma samples were measured.

The cytokine concentrations and chemokine concentrations of the plasma samples were measured by ELISA using the aforementioned ELISA kits. Plates were read at 540 nm with a plate reader (Spectra max 250, Molecular Devices Corporation, Sunnyvale, CA), and the resulting data were analyzed using SOFT max PRO 1.1 (Molecular Devices Corporation, Sunnyvale, CA).

In the aforementioned measurements, a measurement limit of 1.5 pg/mL or less was set for MIP-2, 3.0 pg/mL or less for IL-6, 7.0 pg/mL or less for IL-1β, 1.0 pg/mL or less for INF-γ, 3.0 pg/mL or less for TNF-α, 9.0 pg/mL or less for MCP-1, 0.9 pg/mL or less for IL-10, and 1.0 pg/mL or less for GM-CSF, and values below the measurement limits were given as 0 pg/mL.

The NO concentrations in the plasma were measured by detecting total combined NO₂ and NO₃ using an NO₂/NO₃ assay kit. Each plasma sample was first filtered with a micro-concentrator (Amicon, Beverly, MA, USA) and centrifuged (15000 rpm, 15 minutes, 4°C). NO₃ reductase was added to the centrifuged plasma samples to convert NO₃ to NO₂, and total NO₂ was measured using Gress reagent. Plates were read at 540 nm with a plate reader (Spectra max 250), and the resulting data were analyzed using the aforementioned SOFT max PRO 1:1.

Differences between the control group and the 5'-FMN-Na administration group were analyzed by non-corresponding Student's *t*-test. Statistical analysis was performed using SAS 6.12 (SAS Institute Japan Inc., Tokyo, Japan), and P values of less than 0.05 (two-sided) were considered statistically significant.

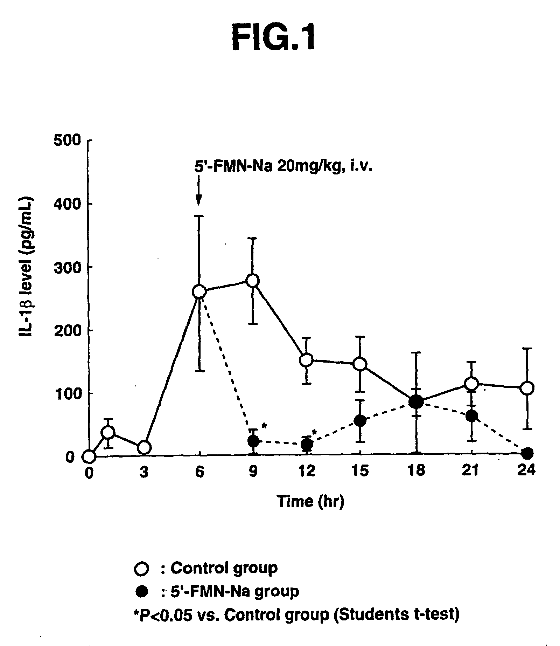

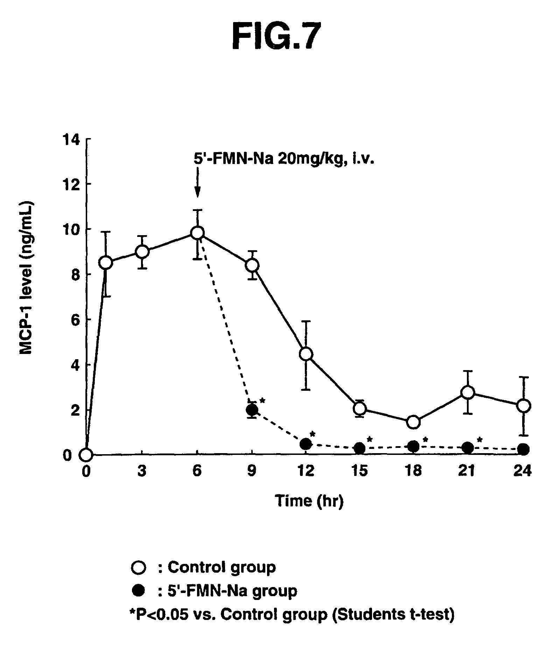

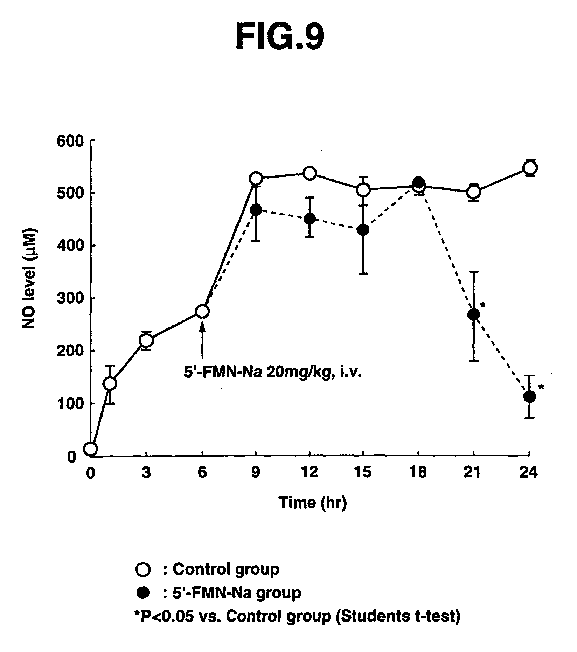

The measurement results for cytokine concentration as measured above are given in Figures 1 through 6, while the measurement results for chemokine concentration are given in Figures 7 through 8 and the measurement results for NO concentration in Figure 9.

In Figures 1 through 9, each plot indicates mean ± standard deviation. The white plots indicate the control group, while the black plots indicate the 5'-FMN-Na administration group.

As shown in Figure 1, IL-1β concentrations in the plasma peaked 9 hours after administration of LPS, and then gradually declined. In the 5'-FMN-Na administration group (5'-FMN-Na administered 6 hours after administration of LPS), however, IL-1β concentrations began to decline immediately after administration of 5'-FMN-Na, and IL-1β concentrations decreased rapidly between 9 and 12 hours after LPS administration.

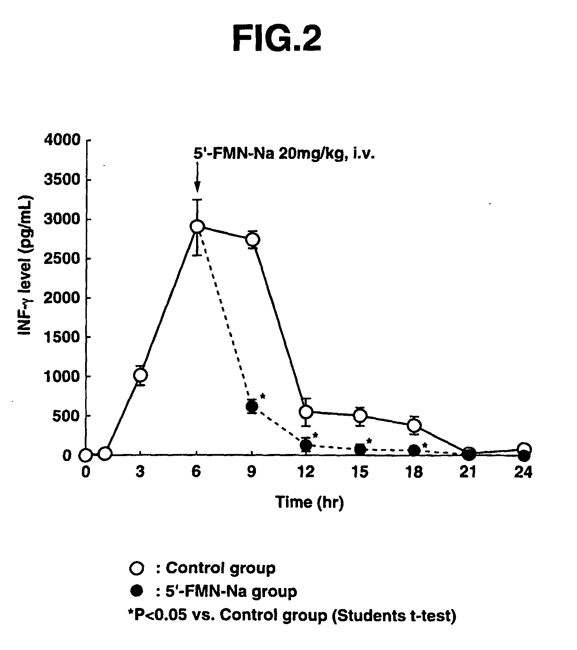

As shown in Figure 2, INF-γ concentrations in the plasma peaked 6 hours after administration of LPS, and then gradually declined. In the 5'-FMN-Na administration group, however, INF-γ concentrations declined rapidly between 9 and 18 hours after LPS administration.

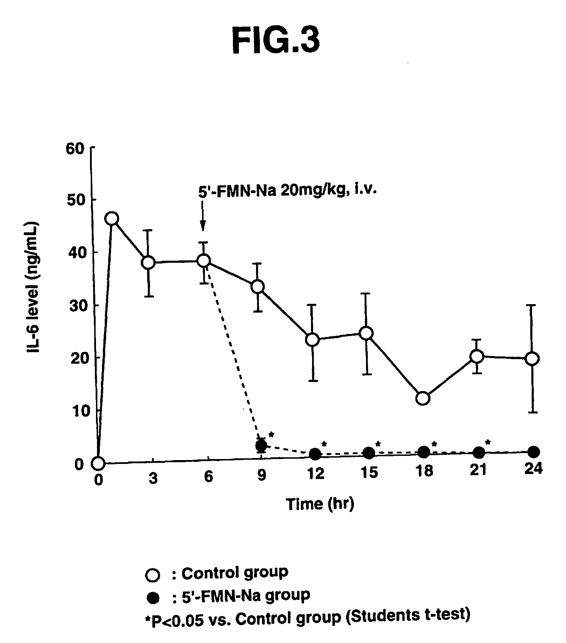

As shown in Figure 3, IL-6 concentrations in plasma rose rapidly after LPS administration, peaking after 1 hour, and then gradually declined. In the control group, IL-6 concentrations were still high 24 hours after LPS administration. In the 5'-FMN-Na administration group, administration of 5'-FMN-Na after 6 hours resulted in a rapid decline in IL-6 concentrations, and this condition was still maintained after 21 hours.

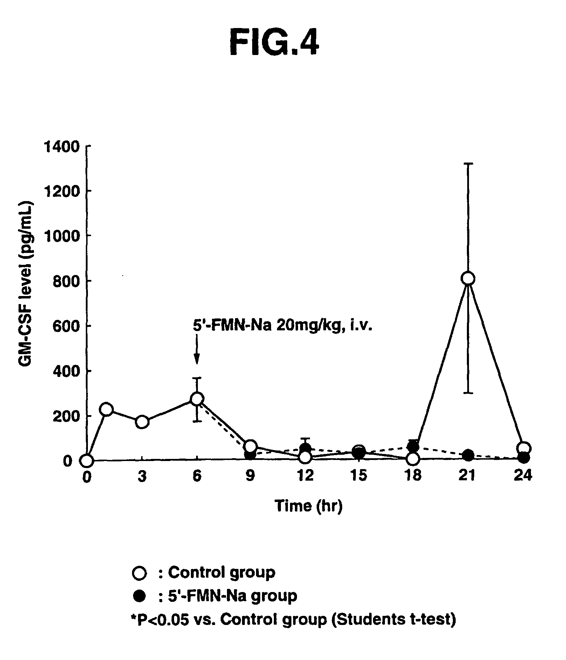

As shown in Figure 4, GM-CSF concentrations in plasma peaked after between 1 and 6 hours, and had declined to their original values after 9 hours. A second peak was observed 21 hours after LPS administration. In the 5'-FMN-Na administration group, however, administration of 5'-FMN-Na after 6 hours tended to depress GM-CSF concentrations 21 hours after LPS administration.

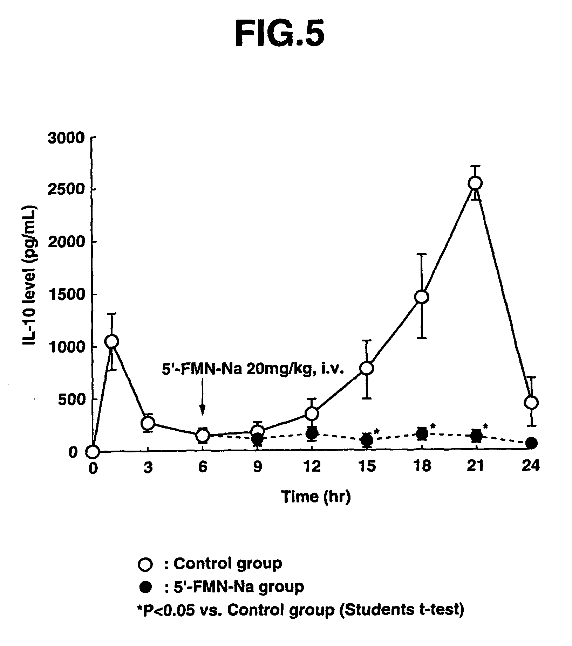

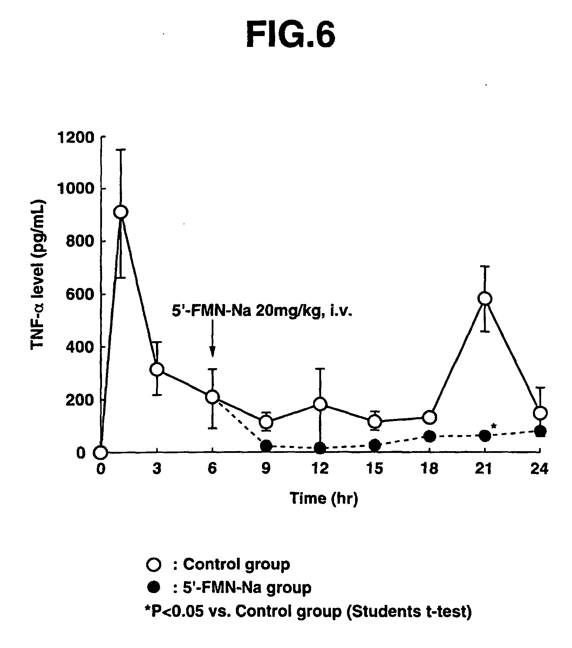

As shown in Figures 5 and 6, as to concentrations of IL-10 and TNF-α, in plasma, each exhibited 2 definitive peaks, one 1 hour after LPS administration and the other 21 hours after. Administration of 5'-FMN-Na depressed IL-10 and TNF-α concentrations at the time of the second peak.

As shown in Figure 7, MCP-1 concentrations in plasma rose rapidly after administration of LPS, peaking after 6 hours. MCP-1 concentrations then declined gradually. Administration of 5'-FMN-Na depressed MCP-1 concentrations, and this state was maintained until 21 hours after LPS administration.

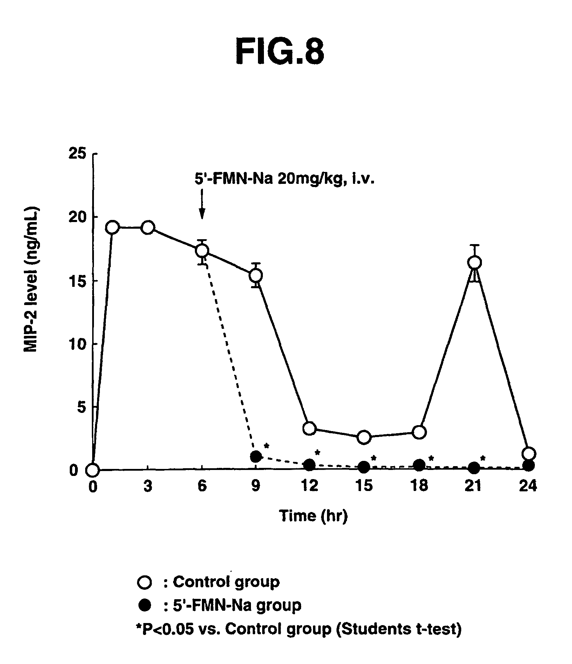

As shown in Figure 8, MIP-2 concentrations in plasma rose rapidly after administration of LPS, peaking after between 1 and 3 hours, and then declined. A second peak was then observed in the control group after 21 hours, but in the 5'-FMN-Na administration group MIP-2 concentrations had declined after 9 hours and this state was maintained after 21 hours.

As shown in Figure 9, NO concentrations rose rapidly after administration of LPS, peaking after 9 hours. These high NO levels were still high after 24 hours. The increase in NO levels is believed to be related to cytokine induction. 5'-FMN-Na had no suppression effect on NO levels between 9 and 18 hours, but had a suppression effect on NO levels after between 21 and 24 hours.

As shown above, 5'-FMN-Na has the effect of suppressing endotoxin-induced increases in blood cytokine, chemokine and NO concentrations.

### [Example 2: Effects on exotoxin-induced shock mouse model]

Male BALB/c mice were purchased at aged 5 weeks from Charles River Japan, and used in the tests after adapting for 1 week under the same conditions as the aforementioned male ICR mice.

0.75 mg/kg of SEB and 1.8 g/kg of D-galactosamine were administered intraperitoneally to the male BALB/c mice (aged 6 weeks) to prepare exotoxin (SEB)-induced shock mice.

In the control group, blood was collected 6 hours, 9 hours, 12 hours, 15 hours and 18 hours after SEB administration (hour 0). In the 5'-FMN-Na group, a single injection of 20 mg/kg of 5'-FMN-Na was administered intravenously 6 hours after administration of SEB, and blood was collected 9 hours, 12 hours, 15 hours and 18 hours after administration of SEB. At each blood collection point each group consisted of 5 mice. Mice that died during the tests were not used as data. Blood was collected from abdominal veins using plastic syringes containing EDTA. Plasma was isolated by centrifugation (3000 rpm, 10 minutes, 4°C) from the collected blood and stored at -80°C.

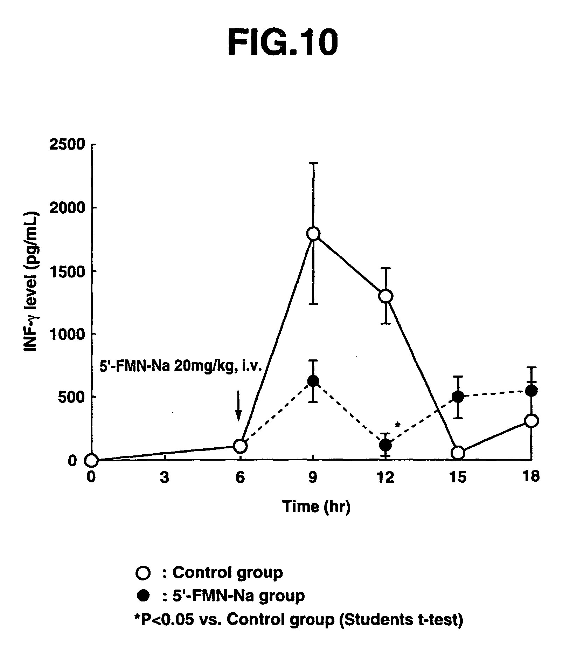

Next, the cytokine concentrations and chemokine concentrations of the various collected plasma samples were measured, analyzed and plotted in the same was as in Example 1. The measurement results for cytokine concentrations and chemokine concentrations are shown in Figures 10 through 12.

As shown in Figure 10, INF-γ concentrations in plasma peaked at 9 hours after administration of SEB, and then declined. However, when 5'-FMN-Na was administered after 6 hours, a suppression effect on INF-γ concentrations was confirmed after about 12 hours.

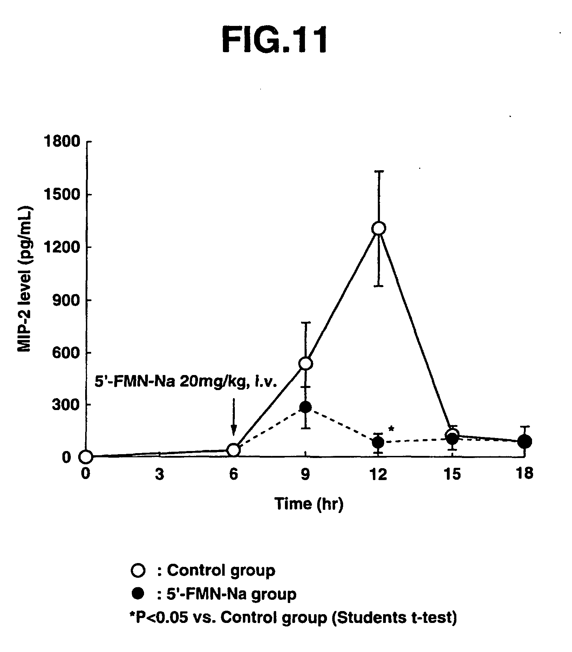

As shown in Figure 11, MIP-2 concentrations in plasma peaked 12 hours after administration of SEB, and then declined. However, when 5'-FMN-Na was administered 6 hours after administration of SEB, an MIP-2 suppression effect was confirmed after about 12 hours.

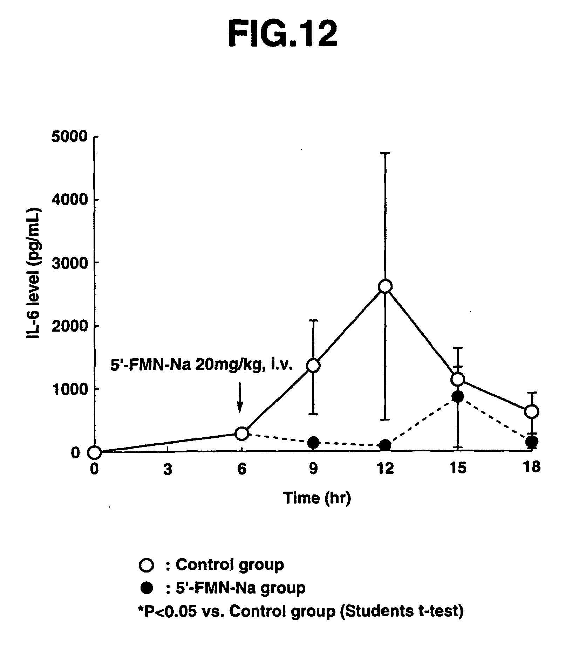

As shown in Figure 12, IL-6 concentrations peaked 12 hours after administration of SEB. When 5'-FMN-Na was administered 6 hours after administration of SEB, a suppression effect on IL-6 concentrations was confirmed after about 9 to 12 hours.

As shown above, it was confirmed that 5'-FMN-Na has the effect of suppressing exotoxin-induced rises in blood cytokine and chemokine concentrations.

### [Example 3: Effects on TNF-α-induced shock mouse model]

Female ICR mice were purchased at aged 4 weeks from Japan CRJ Inc. (Kanagawa, Japan). Male BALB/c mice were purchased at aged 5 weeks from Japan SLC Inc. (Shizuoka, Japan). These mice were kept in a 12-hour light/dark cycle (lights on at 7:00 a.m., lights off at 7:00 p.m.) under conditions of 23°C (permissive range: 20 to 26°C), relative humidity 55% (permissive range: 40 to 70%). The mice were allowed free access to sterile tap water and a laboratory diet (MF, Oriental Yeast Co., Ltd., Tokyo, Japan). They were used in the tests after adapting to this environment for 1 week.

First, 3 µg of human TNF-α and 18 mg of D-galactosamine (0.2 mU30 g body weight) were injected intraperitoneally into female ICR mice (5 weeks old), 10 mice per group. Immediately after administration of TNF-α and D-galactosamine, the mice were given intravenous injections of 20 mg/kg 5'-FMN-Na (5'-FMN-Na administration group) or saline (control group). The survival rate (%) of the mice 7 days after administration of TNF-α was observed. The results are shown in Table 1.

Analysis in Example 3 was performed by Steel test. SAS 6.12 was used as in Example 1, and P values of less than 0.05 (two-sided) were considered statistically significant.

**[Table 1 ]**

| Test group | Survival number | Survival rate (%) |
|---|---|---|
| Saline | 2/10 | 20 |
| 5'-FMN-Na 20 mglkg | 7/10 | 70 |

As shown in Table 1, the survival rate of the control group was 20% while the survival rate of the 5'-FMN-Na administration group was 70%. All of the 8 control group mice died within 3 days.

Next, 3 µg of human TNF-α and 18 g of D-galactosamine (0.2 mU30 g body weight) were injected intraperitoneally into male BALB/c mice (6 weeks old), 10 mice per group. Immediately after administration of TNF-α and D-galactosamine, the mice were given intravenous injections of 20 mg/kg 5'-FMN-Na (5'-FMN-Na administration group) or saline (control group). The survival rate (%) of the mice 7 days after administration of TNF-α was observed. The results are shown in Table 2.

**[Table 2]**

| Test Group | Survival number | Survival rate (%) |
|---|---|---|
| Saline | 1/10 | 10 |
| 5'-FMN-Na 20 mglkg | 6/10 | 60* |

| | | |
|---|---|---|
| *P < 0.05 vs. Saline (Steel test) | | |

As shown in Table 2, the survival rate of the control group was 10%, while the survival rate of the 5'-FMN-Na administration group was 60%. All of the 9 control group mice died within 3 days.

These results reveal that administration of 5'-FMN-Na dramatically improves survival in response to TNF-α-induced shock. In other words, it was demonstrated that administration of 5'-FMN-Na significantly improves survival in response to not only toxin-induced shock but also TNF-α-induced shock.

Next, the effects of addition of 5'-FMN-Na on TNF-α-induced IL-6 production by mouse peritoneal macrophages were investigated. Male ICR mice were sacrificed by bleeding from the carotid artery under ether anesthesia, and after intraperitoneal injection of 4.5 mL/mouse Hanks' solution (HBSS; Gibco Laboratories, Grand Island, NY, USA), peritoneal fluid was collected. The collected peritoneal fluid was centrifuged (1000 rpm, 10 minutes) together with chilled HBSS, and peritoneal macrophages were isolated.

These cells were suspended 1 x 10⁶ cells/mL in Eagles medium (Eagles minimal essential medium; Nissui Pharmaceutical Co., Tokyo, Japan) with 15% inactivated mouse serum added, and cultured in a Lab-Tek chamber (Nalgen Nunc International, Naperville, IL, USA) at 37°C in the presence of 5% CO₂. The mouse peritoneal macrophages were reacted with 10 ng/mL of TNF-α in the presence of 1.56 µg/mL or 25µg/mL of 5'-FMN-Na or without 5'-FMN-Na, and cultured for 24 hours.

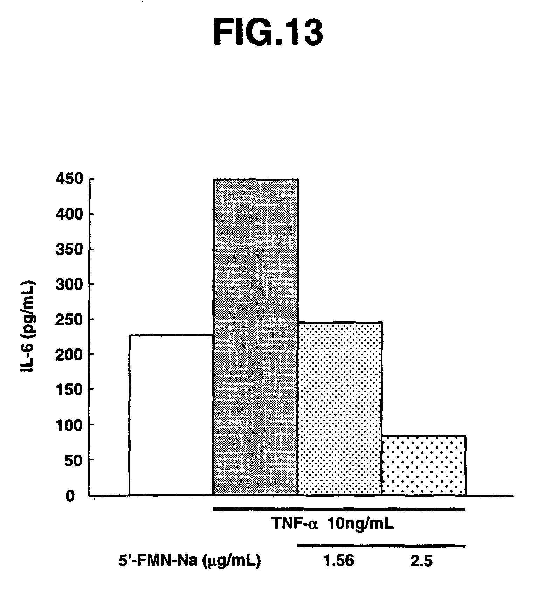

IL-6 was measured using the aforementioned ELISA kit. The measurement results are shown in Figure 13.

As shown in Figure 13, IL-6 levels rose in the case of mouse peritoneal macrophages to which 10 ng/mL of TNF-α was added. In contrast, the induced IL-6 was suppressed in the case of the mouse peritoneal macrophages to which an additional 1.56 µg/mL or 25 µg/mL of 5'-FMN-Na was added.

### [Example 4: Effects on AAPH-intoxication mouse model]

In Example 4, the same male ICR mice were purchased as in Example 1 and adapted under the same conditions.

125 mg/kg of AAPH (0.2 mU30 g body weight) was injected intraperitoneally into male ICR mice (age 6 weeks), 10 mice per group. 24 hours before injection of AAPH, immediately after injection and 1 hour thereafter, the mice were given intravenous injections of 20 mg/kg 5'-FMN-Na (5'-FMN-Na administration group) or saline (control group). The mouse survival rates (%) 7 days after administration of AAPH were observed. The results are shown in Table 3.

In Example 4 the difference between the 5'-FMN-Na group and the control group was analyzed by Steel test. Statistical analysis was performed using SAS 6.12 as in Example 1, and P values of less than 0.05 (two-sided) were considered statistically significant.

**[Table 3]**

| Test Group | Survival number | Survival rate (%) |
|---|---|---|
| Saline | 2/10 | 20 |
| 5'-FMN-Na 20 mg/kg i.v. (-24h, 0, +1h) | 6/10 | 60 |

As shown in Table 3, the survival rate of the control group was 20%, while the survival rate of the 5'-FMN-Na group was 60%. All of the 8 control group mice died within 2 days.

Next, 5'-FMN-Na or saline was administered 24 hours before AAPH injection and immediately after injection as above except that injection was intraperitoneal rather than intravenous, and mouse survival rates (%) 7 days after administration of AAPH were observed, with the results shown in Table 4.

**[Table 4]**

| Test Group | Survival number | Survival rate (%) |
|---|---|---|
| Saline | 1/10 | 10 |
| 5'-FMN-Na 20 mg/kg i.p. (-24h, 0) | 7/10 | 70* |

| | | |
|---|---|---|
| * P < 0.05 vs. Saline (Steel test) | | |

As shown in Table 4, the survival rate of the control group was 10% while the survival rate of the 5'-FMN-Na group was 70%. All of the 9 control group mice died within 3 days.

These results reveal that administration of 5'-FMN-Na dramatically improves survival in response to AAPH poisoning. In other words, since AAPH is a free radical initiator, administration of 5'-FMN-Na has the effect of preventing functional damage to tissues from active oxygen.

Moreover, it is also known that TNF-α increases active oxygen in damaged parts of cell membrane receptors and cell sections (Chandel NS, Trzyna WC, McClintock DS and Schumacker, PT: "Role of oxidants in NF-kappa B activation and TNF-alpha gene transcription induced by hypoxia and endotoxin," *J Immunol* 15, 1013, 1021 (2000)), which indicates that one mechanism of the TNF-α suppression effect of 5'-FMN-Na is a scavenging action against active oxygen.

### [Example 5: Effect on active oxygen production in endothelial cells under high glucose load]

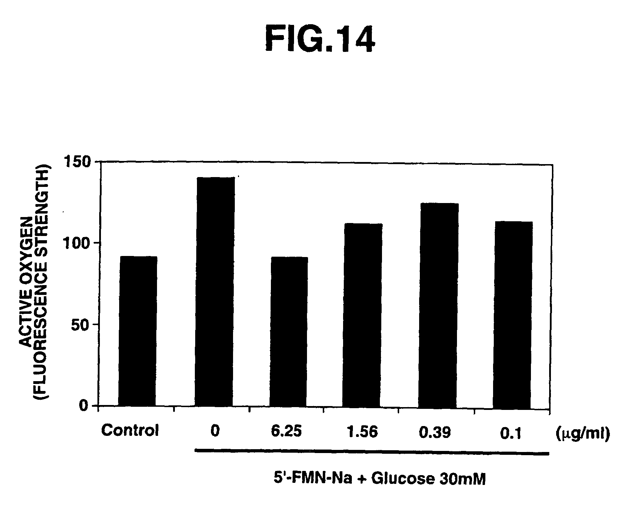

5'-FMN-Na was added to concentrations of 6.25 µg/mL, 1.56 µg/mL, 0.39 µg/mL and 0.1 µg/mL to human vascular endothelial cells (HUVEC) cells cultured on plastic plates, and the cells were CO₂ incubated for 3 hours at 37°C. Next, glucose was added to a glucose concentration of 30 mM on each plate, and the cells were CO₂ incubated for 1 hour at 37°C. After completion of culture the cells were collected and intracellular active oxygen was measured by FACS Calibur (Flow Cytometry System; Becton Dickinson Japan) using BURSTTEST (intracellular active oxygen measurement kit; ORPEGEN Pharma). Specifically, the active oxygen of 10,000 cells was measured from mean fluorescent strength of FL1 using FACS Calibur. The results are shown in Figure 14.

As shown in Figure 14, the fluorescent strength (mean fluorescence) of the control group was 91.2, but under a 30 mM glucose load active oxygen was produced and fluorescent strength rose to 140.5. On the other hand, when 6.25 µg/mL of 5'-FMN-Na was added fluorescent strength fell to 89.5, and the production of active oxygen was suppressed. Moreover, fluorescent strength was 111.9 with 1.56 µg/mL added, 125.0 with 0.39 µg/mL added and 114.2 with 0.1 µg/mL added.

These results reveal that addition of 5'-FMN-Na suppresses production of active oxygen due to high-concentration glucose load.

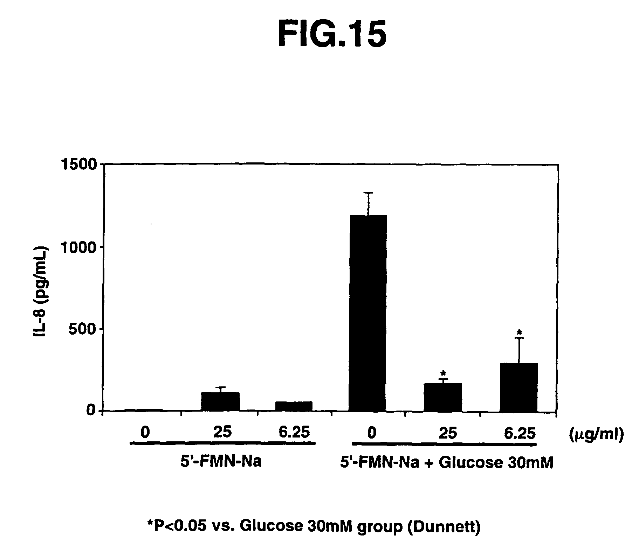

In addition, 5'-FMN-Na was added to concentrations of 25 µg/mL and 6.25 µg/mL to HUVEC cells cultured on plates, and CO₂ incubation performed for 3 hours at 37°C. Next, glucose was added to each plate to a glucose concentration of 30 mM, and CO₂ incubation performed for 2 hours at 37°C. After completion of culture, IL-8 in the culture liquid was measured with an ELISA kit. The results are shown in Figure 15.

As shown in Figure 15, in the control group IL-8 was produced under a glucose load of 30 mM, with IL-8 concentration in the medium rising to 1196 pg/mL. When 25 µg/mL of 5'-FMN-Na was added the IL-8 concentration in the medium fell to 168, while when 6.25 µg/mL was added the IL-8 concentration in the medium fell to 297 µg/mL, so that IL-8 production was significantly suppressed (Dunnett test).

These results reveal that addition of 5'-FMN-Na suppresses IL-8 production due to high-concentration glucose load. The results of Example 5 suggest that a medicament containing 5'-FMN-Na as an active ingredient would act as a therapeutic or preventative agent for diabetes and pathologies of aggravated diabetes.

### INDUSTRIAL APPLICABILITY

The medicament according to the present invention has excellent cytokine suppressing effect, and is useful as a preventative or therapeutic agent or the like for inflammatory conditions accompanied by hypercytokinemia.

## Claims

1. A medicament having cytokine suppression effect, comprising as an active ingredient at least one of riboflavin, a riboflavin derivative or a pharmacologically acceptable salt thereof.

2. The medicament according to Claim 1, which is useful for preventing or treating hypercytokinemia.

3. The medicament according to Claim 1, which is useful for preventing or treating systemic inflammatory response syndrome.

4. The medicament according to Claim 3, wherein said systemic inflammatory response syndrome is not caused by an infection.

5. The medicament according to Claim 4, wherein said systemic inflammatory response syndrome is caused by a wound, a bum, pancreatitis, liver damage, ischemia/reperfusion injury or surgery.

6. The medicament according to any one of Claims 1 through 5, wherein said cytokine suppression effect suppresses at least one of IL-1β, IL-6, IL-10, INF-γ, TNF-α, GM-CSF, IL-8 or MCP-1.

7. A medicament useful for preventing or treating systemic inflammatory response syndrome not caused by an infection, comprising as an active ingredient at least one of riboflavin, a riboflavin derivative or a pharmacologically acceptable thereof.

8. The medicament according to Claim 7, wherein said systemic inflammatory response syndrome is caused by a wound, a bum, pancreatitis, liver damage, ischemia-reperfusion injury or surgery.

9. The medicament according to Claim 1, wherein said riboflavin derivative or pharmacologically acceptable salt thereof is flavin mononucleotide, flavin adenine dinucleotide, riboflavin tetrabutylate, riboflavin sodium phosphate, riboflavin phosphate monodiethanolamine salt, leucoflavin, monohydroflavin, leucoflavin phosphoric acid ester, leucoflavin mononucleotide, leucoflavin adenine dinucleotide or a pharmacologically acceptable salt thereof.

10. The medicament according to Claim 1, wherein at least one of the riboflavin, riboflavin derivative or pharmacologically acceptable salt thereof is contained in the form of an injection, tablet, granule, powder, subtle granule, capsule, pill or oral liquid.

11. The medicament according to Claim 1, wherein said cytokine suppression effect has as one mechanism the scavenging action of active oxygen.

12. A use of at least one of riboflavin, a riboflavin derivative or a pharmacologically acceptable salt thereof for preparation of a preventative or a therapeutic agent for hypercytokinemia.

13. The use of at least one of riboflavin, a riboflavin derivative or a pharmacologically acceptable salt thereof as a preventative or therapeutic agent for hypercytokinemia.

14. A cytokine suppressing agent, comprising as an active ingredient at least one of riboflavin, a riboflavin derivative or a pharmacologically acceptable salt thereof.

15. A method for preventing or treating hypercytokinemia, comprising administering to a subject an effective dose of at least one of riboflavin, a riboflavin derivative or a pharmacologically acceptable salt thereof.
